(19) **Europäisches Patentamt**
**European Patent Office**
**Office européen des brevets**

(11) **EP 1 595 560 B1**

(12) **EUROPÄISCHE PATENTSCHRIFT**

(45) Veröffentlichungstag und Bekanntmachung des Hinweises auf die Patenterteilung:
**30.09.2009 Patentblatt 2009/40**

(51) Int Cl.:
*A61M 1/34* *(2006.01)*    *A61M 1/36* *(2006.01)*

(21) Anmeldenummer: **05009278.2**

(22) Anmeldetag: **28.04.2005**

(54) **Verfahren und Vorrichtung zur Überwachung der Zufuhr von Substitutionsflüssigkeit während einer extrakorporalen Blutbehandlung**

Method and device for supervising the supply of substitution fluid during an extracorporeal blood treatment

Méthode et dispositif pour surveiller l'adduction de liquide de substitution pendant un traitement extracorporel du sang

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **11.05.2004 DE 102004023080**

(43) Veröffentlichungstag der Anmeldung:
**16.11.2005 Patentblatt 2005/46**

(60) Teilanmeldung:
**09009766.8**

(73) Patentinhaber: **Fresenius Medical Care Deutschland GmbH**
**61352 Bad Homburg v.d.H. (DE)**

(72) Erfinder: **Kopperschmidt, Pascal Dr.**
**97456 Dittelbrunn (DE)**

(74) Vertreter: **Luderschmidt, Schüler & Partner Patentanwälte**
**John-F.-Kennedy-Strasse 4**
**65189 Wiesbaden (DE)**

(56) Entgegenhaltungen:
**EP-A- 1 348 458        WO-A-00/51664**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung bezieht sich auf ein Verfahren zur Überwachung der Zufuhr von Substitutionsflüssigkeit für eine Vorrichtung zur extrakorporalen Blutbehandlung mit einem extrakorporalen Blutkreislauf, der eine erste Kammer eines durch eine Membran in die erste Kammer und eine zweite Kammer unterteilten Dialysators oder Filters einschließt, und einem Flüssigkeitssystem, das die zweite Kammer des Dialysators oder Filters einschließt. Darüber hinaus betrifft die Erfindung eine derartige Vorrichtung zur extrakorporalen Blutbehandlung mit einer Einrichtung zur Detektion der Zufuhr von Substitutionsflüssigkeit stromauf oder stromab des Dialysators oder Filters.

Zur Entfernung von harnpflichtigen Substanzen und zum Flüssigkeitsentzug werden beim chronischen Nierenversagen verschiedene Verfahren zur extrakorporalen Blutbehandlung bzw. reinigung eingesetzt. Bei der Hämodialyse wird das Blut des Patienten außerhalb des Körpers in einem Dialysator gereinigt. Der Dialysator weist eine Blutkammer und eine Dialysierflüssigkeitskammer auf, die von eine semipermeablen Membran getrennt sind. Während der Behandlung strömt das Blut des Patienten durch die Blutkammer. Um das Blut effektiv von harnpflichtigen Substanzen zu reinigen, wird die Dialysierflüssigkeitskammer kontinuierlich von frischer Dialysierflüssigkeit durchströmt.

Während bei der Hämodialyse (HD) der Transport der kleinen molekularen Substanzen durch die Membran im Wesentlichen durch die Konzentrationsunterschiede (Diffusion) zwischen der Dialysierflüssigkeit und dem Blut bestimmt wird, werden bei der Hämofiltration (HF) im Plasmawasser gelöste Substanzen, insbesondere höhermolekulare Stoffe durch einen hohen Flüssigkeitsstrom (Konvektion) durch die Membran des Dialysators effektiv entfernt. Bei der Hämofiltration fungiert der Dialysator als Filter. Eine Kombination aus beiden Verfahren ist die Hämodiafiltration (HDF).

Bei der Hämo(dia)filtration wird ein Teil des durch die Membran abgezogenen Serums durch eine sterile Substitutionsflüssigkeit ersetzt, die entweder stromauf des Dialysators oder stromab des Dialysators dem extrakorporalen Blutkreislauf zugeführt wird. Die Zufuhr der Substitutionsflüssigkeit stromauf des Dialysators wird auch als Predilution und die Zufuhr stromab des Dialysators als Postdilution bezeichnet.

Es sind Vorrichtungen zur Hämo(dia)filtration bekannt, bei denen die Dialysierflüssigkeit online aus Frischwasser und Konzentrationen und die Substitutionsflüssigkeit online aus der Dialysierflüssigkeit hergestellt werden.

Bei den bekannten Hämo(dia)filtrationsvorrichtungen wird die Substitutionsflüssigkeit dem extrakorporalen Blutkreislauf von dem Flüssigkeitssystem der Maschine über eine Substitutionsflüssigkeitsleitung zugeführt. Bei der Predilution führt die Substitutionsflüssigkeitsleitung zu einer Anschlussstelle an der arteriellen Blutleitung stromauf des Dialysators oder Filters, während bei der Postdilution die Substitutionsflüssigkeitsleitung zu einer Anschlussstelle an der venösen Blutleitung stromab des Dialysators führt. Die Substitutionsflüssigkeitsleitung weist im Allgemeinen einen Konnektor auf, mit dem sie entweder an die venöse oder arterielle Blutleitung angeschlossen werden kann. Zum Unterbrechen der Flüssigkeitszufuhr ist an der Substitutionsflüssigkeitsleitung eine Klemme oder dgl. vorgesehen. Ein derartiges Hämo(dia)filtrationsgerät ist beispielsweise aus der EP-A-0 189 561 bekannt.

Die Überwachung der Blutbehandlung setzt die Kenntnis voraus, ob die Substitutionsflüssigkeit stromauf oder stromab des Dialysators oder Filters dem extrakorporalen Blutkreislauf zugeführt wird. Beispielsweise spielt die Pre- und Postdilution eine Rolle für die auf einer Leitfähigkeitsmessung beruhenden Online-Clearance-Messung (OCM), da die Leitfähigkeit der Dialysierflüssigkeit stromab des Dialysators davon abhängig ist, ob eine Pre- oder Postdilution erfolgt.

Die EP-A-1 348 458 A1 beschreibt ein Verfahren und eine Vorrichtung zur Überwachung der Zufuhr von Substitutionsflüssigkeit für eine extrakorporale Blutbehandlungsvorrichtung. Für die Detektion der Zufuhr von Substitutionsflüssigkeit stromauf oder stromab des Dialysators oder Filters wird die Laufzeit der Druckwellen einer in der Substitutionsflüssigkeitsleitung angeordneten Substituatpumpe gemessen. Die Zufuhr von Substitutionsflüssigkeit stromauf oder stromab des Dialysators oder Filters wird auf der Grundlage der Laufzeitmessung erkannt. Das bekannte Verfahren setzt den Einsatz einer Druckwellen erzeugenden Substituatpumpe voraus.

Die DE 101 15 991 C1 beschreibt eine Vorrichtung zum Erkennen von Stenosen in einem Schlauchleitungssystem. Die Druckschrift schlägt vor, auf eine Stenose (Engstelle) im Schlauchleitungssystem bei einer Änderung des Frequenzspektrums einer sich in dem Schlauchleitungssystem ausbreitenden oszillierenden Drucksignals zu schließen. Das Funktionsprinzip der bekannten Vorrichtung beruht darauf, dass die Ursache für die Änderung des dynamischen Verhaltens des Schlauchleitungssystems die Compliance des Leitungssystems ist, d. h. das elastische Nachgeben unter Druck.

Der Erfindung liegt die Aufgabe zu Grunde, ein Verfahren anzugeben, das die Erkennung von Pre- und Postdilution mit hoher Zuverlässigkeit erlaubt. Darüber hinaus ist eine Aufgabe der Erfindung, eine Vorrichtung zur extrakorporalen Blutbehandlung anzugeben, die über eine Einrichtung verfügt, mit der Pre- und Postdilution zuverlässig erkannt werden kann.

Die Lösung dieser Aufgaben erfolgt erfindungsgemäß mit den in den Patentansprüchen 1 bzw. 7 angegebenen Merkmalen.

Das erfindungsgemäße Verfahren und die erfindungsgemäße Vorrichtung beruhen auf einer Messung des Drucks im Flüssigkeitssystem stromab des Dialysators oder Filters.

Bei dem erfindungsgemäßen Verfahren und der erfindungsgemäßen Vorrichtung wird die Substitutionsflüssigkeit fördernde Substituatpumpe abgeschaltet und/oder eingeschaltet. Der Erfinder hat erkannt, dass die Veränderung des Drucks nach dem Abschalten und/oder Einschalten der Substituatpumpe einen charakteristischen Verlauf hat. Die Zufuhr von Substitutionsflüssigkeit stromauf oder stromab des Dialysators oder Filters wird auf der Grundlage der Veränderung des Drucks nach dem Abschalten oder Einschalten der Substituatpumpe erkannt. Eine für die Veränderung des Drucks charakteristische Größe ist ein plötzlicher Druckanstieg und/oder Druckabfall.

Grundsätzlich kann die Erkennung nur auf der Veränderung des Drucks nach dem Abschalten oder Einschalten der Substituatpumpe erfolgen. Eine höhere Zuverlässigkeit ist aber dann gegeben, wenn die Erkennung von Pre- oder Postdilution auf der Grundlage der Veränderung des Drucks sowohl beim Abschalten als auch Einschalten der Substituatpumpe oder umgekehrt erfolgt.

Bei einer bevorzugten Ausführungsform, die eine Erkennung von Pre- und Postdilution mit besonders hoher Zuverlässigkeit erlaubt, wird die bereits eingeschaltete Substituatpumpe abgeschaltet und nach Ablauf eines vorgegebenen Zeitintervalls wieder eingeschaltet, wobei der Druck stromab des Dialysators oder Filters gemessen wird. Eine Zufuhr von Substitutionsflüssigkeit stromauf oder stromab des Dialysators oder Filters wird auf der Grundlage des Wechsels zwischen einem vorausgehenden Druckanstieg und einem nachfolgenden Druckabfall oder zwischen einem vorausgehenden Druckabfall und einem nachfolgenden Druckanstieg erkannt.

Wenn eine Abfolge von einem Druckanstieg und einem Druckabfall detektiert wird, wird auf die Zufuhr von Substitutionsflüssigkeit stromab des Dialysators oder Filters geschlossen, während auf die Zufuhr von Substitutionsflüssigkeit stromauf des Dialysators oder Filters geschlossen wird, wenn eine Abfolge von einem Druckabfall und einem Druckanstieg detektiert wird.

Ein Vorteil liegt darin, dass keine Druckpulse ausgewertet werden, die sich im Blutkreislauf fortpflanzen. Daher ist es nicht erforderlich, eine oszillierende Druckpulse erzeugende Substituatpumpe vorzusehen, um zwischen postdilutiver und predilutiver Substitution unterscheiden zu können.

Zur Unterdrückung von Störsignalen wird das Drucksignal vorzugsweise mit einem Tiefpassfilter gefiltert.

Die charakteristische Pulsfolge nach Stop und Start der Substituatpumpe kann auf der Grundlage eines Vergleichs des Drucksignals im Blutkreislauf stromab des Dialysators oder Filters mit einem oberen und unteren Grenzwert erfolgen, der für einen auf die Pre- oder Postdilution zurückzuführenden Druckanstieg oder Druckabfall charakteristisch ist.

Die Einrichtung zur Detektion der Zufuhr von Substitutionsflüssigkeit stromauf oder stromab des Dialysators oder Filters der erfindungsgemäßen extrakorporalen Blutbehandlungsvorrichtung weist eine Steuereinheit zum Abschalten und/oder Einschalten der Substituatpumpe, eine Messeinheit zum Messen des Drucks im Blutkreislauf stromab des Dialysators oder Filters und eine Auswerteinheit auf. Die Auswerteinheit ist derart ausgebildet, dass eine Zufuhr von Substitutionsflüssigkeit stromauf oder stromab des Dialysators oder Filters auf der Grundlage der Veränderung des venösen Drucks nach dem Abschalten und/oder Einschalten der Substituatpumpe erkannt wird.

Die Messeinheit zum Messen des venösen Drucks weist vorzugsweise einen venösen Drucksensor und einen mit dem Signalausgang des Drucksensors verbundenen Tiefpassfilter auf.

Die Auswerteinheit weist vorzugsweise einen Komparator auf, der das Ausgangssignal des Drucksensors zur Detektion eines Druckanstiegs und/oder Druckabfalls mit einem vorgegebenen oberen und/oder unteren Grenzwert vergleicht.

Das erfindungsgemäße Verfahren und die erfindungsgemäße Vorrichtung finden in vorteilhafter Weise bei der Bestimmung der Dialysedosis mittels des sog. Online-Clearance-Monitoring (OCM) und bei eine Substituatvorgabe in Abhängigkeit von dem Blutfluss Verwendung. Das Verfahren und die Vorrichtung liefern darüber hinaus eine Entscheidungshilfe in allen relevanten die Dialyse betreffenden Parametern, wenn eine Unterscheidung zwischen postdilutiver- und predilutiver Substituatgabe notwendig ist. Das Verfahren und die Vorrichtung können auch Anwendung finden bei der Bestimmung des Blutflusses, der Bestimmung der Shunt- oder Fistelrezirkulation, in der Überwachung des relativen Blutvolumens bzw. Hämatokrits, der Ermittlung der Füllvolumina des Dialysators oder Filters sowie der Erkennung des venösen Nadeltyps.

Die meisten für die Detektionseinrichtung erforderlichen Komponenten sind im Allgemeinen in den bekannten Blutbehandlungsvorrichtungen bereits vorhanden. Beispielsweise kann auf den venösen Drucksensor zum Messen des Drucks im Blutkreislauf stromab des Dialysators oder Filters zurückgegriffen werden. Auch steht eine Mikroprozessorsteuerung zur Verfügung. Damit ist der apparative Aufwand relativ gering.

Außer der Messeinheit zum Messen des Drucks im extrakorporalen Kreislauf stromab des Dialysators oder Filters, die im allgemeinen Bestandteil der bekannten Blutbehandlungsvorrichtungen ist, werden weitere Hardware-Komponenten nicht benötigt.

Im Folgenden wird ein Ausführungsbeispiel des erfindungsgemäßen Verfahrens sowie der erfindungsgemäßen Blutbehandlungsvorrichtung unter Bezugnahme auf die Figuren näher erläutert.

Es zeigen:

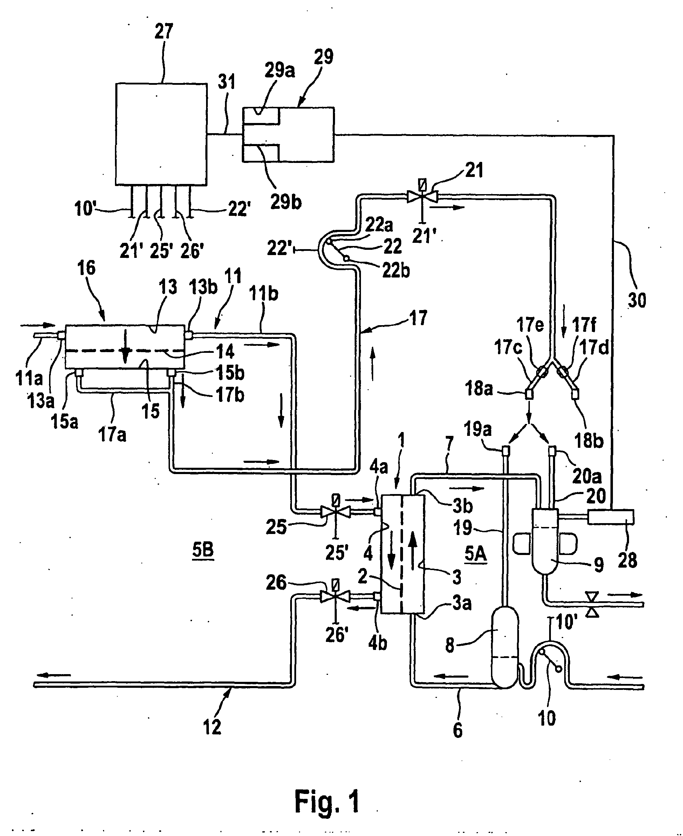

Figur 1     eine Ausführungsform einer erfindungsgemäßen Vorrichtung zur extrakorporalen Blutbehandlung mit einer Einrichtung zur Detektion von Pre- und Postdilution in stark

| Figur 2 A | eine prinzipielle Darstellung des zeitlichen Verlaufs des Drucks im extrakorporalen Blutkreislauf stromab des Dialysators oder Filters bei Postdilution, |
| Figur 2 B | eine prinzipielle Darstellung des zeitlichen Verlaufs des venösen Drucks bei Predilution und |
| Figur 3 A | den während einer in vitro HDF-Dialysebehandlung gemessenen mittleren venösen Druck als Funktion der Zeit bei Postdilution, |
| Figur 3 B | den mittleren venösen Druck bei Predilution. |

Figur 1 zeigt eine vereinfachte schematische Darstellung der wesentlichen Komponenten einer Hämo(dia)filtrationsvorrichtung zusammen mit einer Einrichtung zur Detektion der Zufuhr von Substitutionsflüssigkeit in den extrakorporalen Blutkreislauf stromauf oder stromab des Dialysators oder Filters. Wenn nachfolgend von einem Dialysators die Rede ist, wird darunter auch ein Filter verstanden.

Die Hämo(dia)filtrationsvorrichtung weist einen Dialysator 1 auf, der durch eine semipermeable Membran 2 in eine von Blut durchflossene erste Kammer 3 und eine von Dialysierflüssigkeit durchflossene zweite Kammer 4 getrennt ist. Die erste Kammer 3 ist in einen extrakorporalen Blutkreislauf 5A geschaltet, während die zweite Kammer 4 in das Flüssigkeitssystem 5B der Hämo(dia)filtrationsvorrichtung geschaltet ist.

Der extrakorporale Blutkreislauf 5A umfasst eine arterielle Blutleitung 6, die zu dem Einlass 3a der Blutkammer 3 führt, und eine venöse Blutleitung 7, die von dem Auslass 3b der Blutkammer 3 des Dialysators 1 abgeht. Zur Eliminierung von Luftblasen sind in die arterielle Blutleitung 6 eine arterielle Tropfkammer 8 und in die venöse Blutleitung 7 eine venöse Tropfkammer 9 geschaltet. Das Blut des Patienten wird durch die Blutkammer des Dialysators mittels einer arteriellen Blutpumpe 10, insbesondere Rollenpumpe gefördert, die an der arteriellen Blutleitung 6 angeordnet ist.

Das Flüssigkeitssystem 5B umfasst eine Dialysierflüssigkeitszuführleitung 11, die zu dem Einlass 4a der Dialysierflüssigkeitskammer 4 führt, und eine Dialysierflüssigkeitsabführleitung 12, die von dem Auslass 4b der Dialysierflüssigkeitskammer 4 des Dialysators 1 abgeht. Über die Dialysierflüssigkeitszuführleitung 11 strömt frische Dialysierflüssigkeit aus einer nicht dargestellten Dialysierflüssigkeitsquelle in die Dialysierflüssigkeitskammer, während die verbrauchte Dialysierflüssigkeit aus der Dialysierflüssigkeitskammer über die Dialysierflüssigkeitsabführleitung 12 zu einem nicht dargestellten Abfluss abgeführt wird. Die in den Hämo(dia)filtrationsvorrichtungen im Allgemeinen vorgesehene Bilanzierungseinrichtung zur Bilanzierung frischer gegen verbrauchte Dialysierflüssigkeit ist der besseren Übersichtlichkeit halber nicht dargestellt. Auch sind zusätzliche Einrichtungen zum Reinigen und Spülen des Systems nicht dargestellt.

Die Dialysierflüssigkeitszuführleitung 11 umfasst einen ersten Abschnitt 11a, der zu dem Einlass 13a einer ersten Kammer 13 eines durch eine Membran 14 in die erste Kammer und eine zweite Kammer 15 unterteilten Sterilfilters 16 führt, und einen zweiten Abschnitt 11b, der von dem Auslass 13b der ersten Kammer 13 des Filters 16 abgeht und zu dem Einlass 4a der Dialysierflüssigkeitskammer 4 führt.

Während der Dialysebehandlung kann z. B. Dialysierflüssigkeit aus dem Flüssigkeitssystem 5B als Substitutionsflüssigkeit über eine Substitutionsflüssigkeitsleitung 17 dem extrakorporalen Blutkreislauf 5A zugeführt werden. Die Flüssigkeitsleitung 17 weist an beiden Enden jeweils zwei Leitungsabschnitte 17a, 17b, 17c, 17d auf. Der Leitungsabschnitt 17a ist mit dem ersten Auslass 15a des Sterilfilters 16 verbunden, während an den Leitungsabschnitten 17c und 17d jeweils ein Konnektor 18a, 18b angeschlossen ist. Mit den beiden Konnektoren 18a, 18b kann die Substitutionsflüssigkeitsleitung 17 an eine zu der arteriellen Tropfkammer 8 führende Anschlussleitung 19 bzw. eine zu der venösen Tropfkammer 9 führende Anschlussleitung 20 angeschlossen werden. Die Anschlussleitungen 19, 20 verfügen dafür über entsprechende Anschlussstücke 19a, 20a. Auf den Schlauchleitungsabschnitten 17c, 17d sitzen Schlauchklemmen 17e, 17f, mit denen wahlweise eine Flüssigkeitsverbindung nur zu der arteriellen oder venösen Tropfkammer 8, 9 hergestellt werden kann. Es ist aber auch möglich, das die Substitutionsflüssigkeitsleitung 17 mit beiden Anschlussleitungen 19, 20 verbunden und beide Schlauchklemmen 17e, 17f offen sind.

Zum Abklemmen der Substitutionsflüssigkeitsleitung 17 ist stromab des Sterilfilters 16 ein Absperrorgan 21, beispielsweise eine Schlauchklemme vorgesehen, die vorzugsweise elektromagnetisch betätigt wird. Die Substitutionsflüssigkeit wird mittels einer Okklusionspumpe, insbesondere Rollenpumpe 22 gefördert, in die die Substitutionsflüssigkeitsleitung 17 eingelegt ist. Derartige Rollenpumpen gehören zum Stand der Technik. Sie verfügen über mehrere Rollen 22a, 22b, mit denen der Querschnitt der Schlauchleitung zur Förderung der Flüssigkeit verringert werden kann. Dadurch entstehen Druckwellen, die sich in beiden Richtungen über die Substitutionsflüssigkeitsleitung fortpflanzen können. Es sei bemerkt, dass eine Druckwellen erzeugende Okklusionspumpe als Substituatpumpe bei der ersten Ausführungsform der erfindungsgemäßen Blutbehandlungsvorrichtung nicht erforderlich ist. Vielmehr kann zur Förderung der Substitutionsflüssigkeit auch eine Substituatpumpe Verwendung finden, die Druckwellen nicht erzeugt. Darin liegt ein Vorteil dieser Ausführungsform.

Zur Abkopplung des Dialysators 1 von dem Flüssigkeitssystem 5B sind in der Dialysierflüssigkeitszuführleitung 11 stromauf und in der Dialysierflüssigkeitsabführleitung 12 stromab des Dialysators 1 jeweils ein elektromagnetisch betätigbares Absperrorgan 25, 26 vorgesehen.

Die Blutpumpe 10, die Substituatpumpe 22 sowie die Absperrorgane 21, 25 und 26 sind über Steuerleitungen 10', 22', 21', 25' und 26' mit einer zentralen Steuer- und Regeleinheit 27 verbunden, von der die einzelnen Komponenten unter Berücksichtigung der vorgegebenen Behandlungsparameter angesteuert werden.

Zum Betrieb der Hämo(dia)filtrationsvorrichtung als Hämodialysevorrichtung wird das Absperrorgan 21 geschlossen, wobei Dialysierflüssigkeit durch die Dialysierflüssigkeitskammer 4 des Dialysators strömt. Zum Betrieb der Hämo(dia)filtrationsvorrichtung als Hämodiafiltrationsvorrichtung wird das Absperrorgan 21 geöffnet, so dass aus dem Sterilfilter 16 sterile Dialysierflüssigkeit als Substitutionsflüssigkeit wahlweise in die arterielle Tropfkammer 8 (Predilution) oder venöse Tropfkammer 9 (Postdilution) strömt. Es ist aber auch ein Betrieb der Hämo(dia)filtrationsvorrichtung nur als Hämofiltrationsvorrichtung möglich, wenn der Zufluss von Dialysierflüssigkeit in die Dialysierflüssigkeitskammer 4 des Dialysators unterbrochen wird. Zur Unterbrechung der Flüssigkeitszufuhr wird das Absperrorgan 25 stromauf des Dialysators geschlossen.

Die Einrichtung zur Detektion von Pre- und Postdilution weist eine Steuereinheit auf, die Teil der zentralen Steuer- und Regeleinheit 27 der Blutbehandlungsvorrichtung ist Darüber hinaus weist die Detektionseinrichtung eine Messeinheit 28 zum Messen des Drucks im extrakorporalen Blutkreislauf stromab des Dialysators 3 und eine Auswerteinheit 29 auf. Als Messeinheit ist ein an der Tropfkammer 9 angebrachter Drucksensor 28 vorgesehen, der ein von dem venösen Druck abhängiges elektrisches Drucksignal erzeugt. Das Drucksignal des Drucksensors 28 wird über eine Datenleitung 30 der Auswerteinheit 29 zugeführt, die wiederum über eine Datenleitung 31 mit der Steuereinheit 27 in Verbindung steht. Die Auswerteinheit 29 weist einen Tiefpassfilter 29a zum Filtern des Drucksignals des Drucksensors 28 und einen Komparator 29b auf, mit dem das Drucksignal mit einem vorgegebenen oberen und unteren Grenzwert verglichen wird.

Nachfolgend wird die Detektion der Zufuhr von Substitutionsflüssigkeit stromauf oder stromab des Dialysators im einzelnen beschrieben. Die Steuereinheit 27 der Detektionseinrichtung schaltet vorzugsweise zu Beginn der Blutbehandlung für die Einleitung der Messung die bereits laufende Substituatpumpe 22 ab. Nach Ablauf eines vorgegebenen Zeitintervalls schaltet die Steuereinheit 27 die Substituatpumpe 22 wieder an. Während die Substituatpumpe ausgeschaltet ist, misst der Drucksensor 28 den venösen Druck. Das mit dem Tiefpassfilter 29a gefilterte venöse Drucksignal des Sensors 28 wird in dem Komparator 29b der Auswerteinheit 29 mit einem oberen und unteren Grenzwert verglichen, um entweder nach dem Abschalten der Substituatpumpe einen Druckanstieg und nach dem Einschalten der Substituatpumpe einen Druckabfall oder nach dem Abschalten der Substituatpumpe einen Druckabfall und nach dem Einschalten der Substituatpumpe einen Druckanstieg zu detektieren.

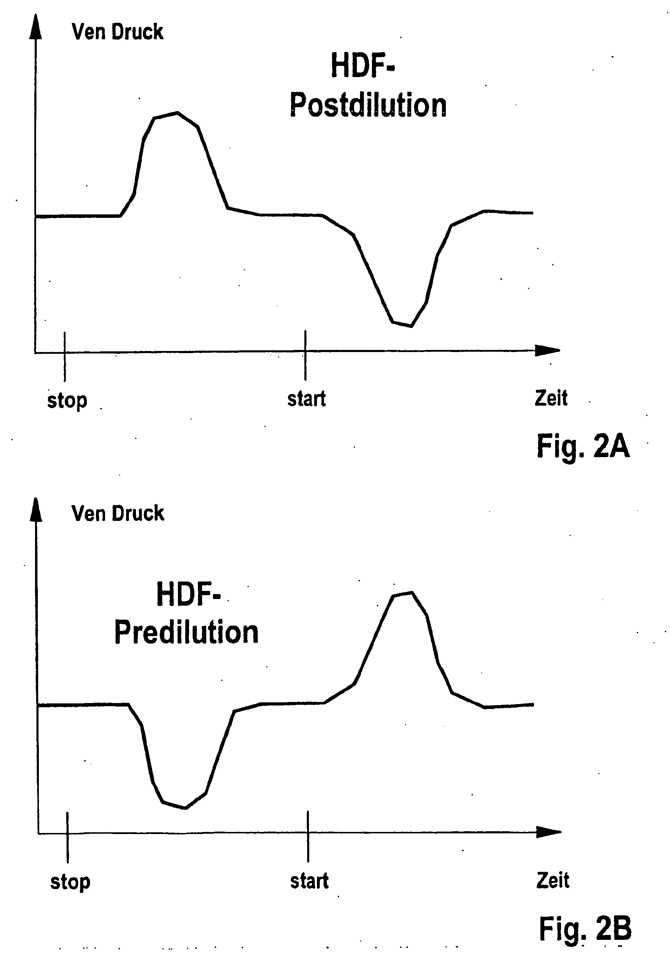

Figur 2A zeigt den prinzipiellen zeitlichen Verlauf des venösen Drucks bei Postdilution und Figur 2B den zeitlichen Verlauf des Drucks bei Predilution. Deutlich ist zu erkennen, dass bei Postdilution der Druck zunächst ansteigt, um anschließend abzufallen. Bei Predilution fällt der Druck zunächst ab, um dann anzusteigen. Auf der Grundlage dieser beiden charakteristischen Verläufe erkennt die Auswerteinheit, ob Postdilution oder Predilution vorliegt. Das Ergebnis kann auf einer nicht dargestellten Anzeigeeinheit optisch und/oder akustisch angezeigt werden.

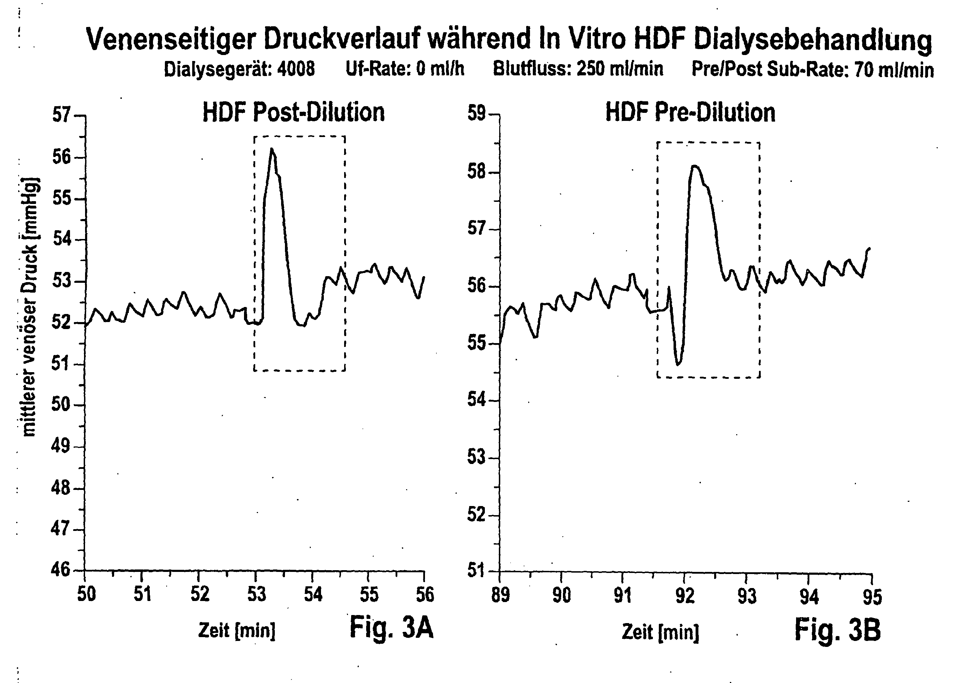

Die Figuren 3A und 3B zeigen den venösen Druck als Funktion der Zeit für den Fall, dass das vorgegebene Zeitintervall, in dem die Substituatpumpe ausgeschaltet ist, relativ lang ist. Wenn ein kürzeres Zeitintervall vorgegebenen wird, liegen die Maxima bzw. Minima näher bei einander. Zur Einleitung der Messung wird die bereits eingeschaltete Substituatpumpe für ein vorgegebenes Zeitintervall ausgeschaltet. Grundsätzlich ist es aber auch möglich, die zunächst ausgeschaltete Substituatpumpe für ein vorgegebenes Zeitintervall einzuschalten.

Der in den Figuren 2A und 2B gezeigte charakteristische Verlauf des Drucksignals ist auf die veränderte Viskosität des Blutes bei Pre- oder Postdilution zurückzuführen. Bei der Postdilution wird die Viskosität des Blutes aufgrund der Zufuhr von Substitutionsflüssigkeit in der venösen Blutleitung 7 stromab der Tropfkammer 9 verringert. Wird die Substituatpumpe 22 angehalten, erhöht sich die Viskosität des Bluts in diesem Abschnitt der venösen Blutleitung. Folglich tritt an der venösen Nadel ein erhöhter Druckabfall auf. Dadurch steigt der venöse Druck an. Wenn die Substituatpumpe wieder eingeschaltet wird, verringert sich die Viskosität des Blutes in der venösen Blutleitung 7 stromab der Tropfkammer 9, so dass der venöse Druck abfällt. Folglich erfolgt nach dem Stop der Substituatpumpe ein Druckpuls mit positivem Vorzeichen und nach dem Start der Pumpe ein Druckpuls mit negativem Vorzeichen. (Fig. 2A). Die Pulsbreite hängt vom Verhältnis des Blutvolumens zwischen der Dialysierflüssigkeitskammer 3 des Dialysators 1 und der Tropfkammer 9 sowie dem Blutfluss ab. Bei Predilution hingegen kehrt sich nach dem Anhalten und Einschalten der Substituatpumpe die Reihenfolge der Vorzeichen des Druckpulses um. Es erfolgt beim Stop der Pumpe ein Druckabfall und nach dem Start der Pumpe ein Druckanstieg (Figur 2B).

Die Figuren 3A und 3B zeigen den mittleren venösen Druck [mmHg] als Funktion der Zeit während einer in vitro HDF-Dialysebehandlung, wobei der Blutfluss auf 250 ml/min, die Substitutionsrate für Pre- und Postdilution auf 70 ml/min und die Ultrafiltrationsrate auf 0 ml/h eingestellt ist. In der Praxis zeigt sich, dass die Amplituden des Druckanstiegs oder Druckabfalls unterschiedlich sind. Der Druckanstieg oder Druckabfall ist aber sowohl bei der Pre- als auch Postdilution deutlich zu erkennen. Die vorgegebenen oberen und unteren Grenzwerte sind derart einzustellen, dass sie unterhalb bzw. oberhalb der Maxima bzw. Minima liegen. Andererseits sollten die oberen und unteren Grenzwerte aber oberhalb bzw. unterhalb der dem Drucksignal überlagerten Druckschwankungen liegen.

Aus den positiven bzw. negativen Druckpulsen kann auch auf die Fistelrezirkulation geschlossen werden. Eine Wiederholung der Druckpulsfolge, die sich in der venösen Blutleitung 7 mit dem venösen Drucksensor 28 nachweisen lässt, in der arteriellen Blutleitung 6, die sich mit einem arteriellen Drucksensor nachweisen lässt, deutet auf eine Rezirkulation hin. Als Maß für die Rezirkulation dient das Verhältnis zwischen dem Integral des Drucksignals des arteriellen Drucksensors in einem vorgegebenen Zeitintervall, in dem der sich wiederholende negative bzw. positive Druckpuls liegt und dem Integral des Drucksignals des venösen Drucksensors 28 in einem vorgegebenen Zeitintervall,

in dem der negative bzw. positive Druckpuls liegt. Die Rezirkulation Rez [%] berechnet sich nach der folgenden Gleichung:

$$\mathrm{Re}\,z[\%] = 100 \cdot \left( \frac{\int p_{an} dt}{\int p_{ven} dt} \right)$$

Aus der Zeitdifferenz ($\Delta t$) zwischen dem Beginn und Ende der Druckpulserhöhung oder Druckpulsverringerung kann bei Kenntnis der Blutförderrate ($Q_b$) auf das Füllvolumen ($V_{PD}$) des Schlauchsegments zwischen dem Eingang 3a der Dialysierflüssigkeitskammer 3 des Dialysators 1 und der Anschlussstelle der Substitutionsflüssigkeitsleitung 17, 20 an der Tropfkammer 9 geschlossen werden. Das Volumen berechnet sich nach der folgenden Gleichung:

$$V_{PD} = Q_b \Delta t = Q_b (t_{Ende} - t_{Beginn})$$

Darüber hinaus kann auf das Füllvolumen ($V_{PD}$) des Schlauchsegments zwischen dem Eingang 3a der Dialysierflüssigkeitskammer 3 und der venösen Nadel geschlossen werden, wenn bei der HDF-Predilution das vorgegebene Zeitintervall zwischen dem Anhalten der Substituatpumpe 22 und dem Beginn des Druckabfalls bzw. das Zeitintervall zwischen dem Einschalten der Substituatpumpe und dem Beginn des Druckanstiegs mit der Blutförderrate ($Q_b$) multipliziert wird. Bei HDF-Postdilution besteht ein analoger Zusammenhang, wobei die Zeitdifferenz zwischen dem Stop der Substituatpumpe und dem Beginn des Druckpulsanstiegs bzw. die Zeitdifferenz zwischen dem Start der Substituatpumpe und dem Beginn des Druckpulsabfalls überwacht wird.

In umgekehrter Weise kann bei Kenntnis des Füllvolumens jeweils der oben genannten Schlauchsegmente und Zeitdifferenzen der wahre Blutfluss ermittelt werden.

Ist der Zusammenhang zwischen Hämatokrit, Blutdruckabfall bzw. Blutdruckanstieg, Blutförderrate und Nadelgeometrie bekannt, so gibt die Amplitude des Druckanstiegs bzw. -abfalls Aufschluss über den Typ der Nadel. Wenn der Typ der Nadel bekannt ist, kann entsprechend auf den Hämatokrit geschlossen werden.

So kann die Änderung des Hämatokrits während der Dialysebehandlung anhand der Änderung der Amplitude der Druckpulse überwacht werden.

**Patentansprüche**

1. Verfahren zur Überwachung der Zufuhr von Substitutionsflüssigkeit für eine Vorrichtung zur extrakorporalen Blutbehandlung mit einem extrakorporalen Blutkreislauf (5A), der eine erste Kammer (3) eines durch eine Membran (2) in die erste Kammer und eine zweite Kammer (4) unterteilten Dialysators (1) oder Filters einschließt, und einem Flüssigkeitssystem (5B), dass die zweite Kammer des Dialysators oder Filters einschließt, wobei Substitutionsflüssigkeit stromauf oder stromab des Dialysators oder Filters mit einer Substituatpumpe (22) zugeführt wird, **dadurch gekennzeichnet, dass** für die Detektion der Zufuhr von Substitutionsflüssigkeit die abgeschaltete Substituatpumpe (22) eingeschaltet und/oder die eingeschaltete Substituatpumpe abgeschaltet wird und dass der Druck im extrakorporalen Blutkreislauf (5A) stromab des Dialysators (1) oder Filters gemessen wird, wobei eine Zufuhr von Substitutionsflüssigkeit stromauf oder stromab des Dialysators oder Filters auf der Grundlage der Veränderung des Drucks nach dem Abschalten und/oder Einschalten der Substituatpumpe erkannt wird.

2. Verfahren nach Anspruch 1, **dadurch gekennzeichnet, dass** für die Detektion der Zufuhr von Substitutionsflüssigkeit die eingeschaltete Substituatpumpe (22) abgeschaltet und nach Ablauf eines vorgegebenen Zeitintervalls die Substituatpumpe eingeschaltet wird, und der Druck im Blutkreislauf (5A) stromab des Dialysators (1) oder Filters gemessen wird, wobei eine Zufuhr von Substitutionsflüssigkeit stromauf oder stromab des Dialysators oder Filters auf der Grundlage des Wechsels zwischen einem vorausgehenden Druckanstieg und einem nachfolgenden Druckabfall oder zwischen einem vorausgehenden Druckabfall und einem nachfolgenden Druckanstieg erkannt wird.

3. Verfahren nach Anspruch 2, **dadurch gekennzeichnet, dass** auf die Zufuhr von Substitutionsflüssigkeit stromab des Dialysators (1) oder Filters geschlossen wird, wenn eine Abfolge von einem Druckanstieg und einem Druckabfall detektiert wird.

4. Verfahren nach Anspruch 2, **dadurch gekennzeichnet, dass** auf die Zufuhr von Substitutionsflüssigkeit stromauf des Dialysators (1) oder Filters geschlossen wird, wenn eine Abfolge von einem Druckabfall und einem Druckanstieg detektiert wird.

5. Verfahren nach einem der Ansprüche 1 bis 4, **dadurch gekennzeichnet, dass** der Druck im Blutkreislauf (5A) stromab des Dialysators (1) oder Filters mit einem Drucksensor (28) gemessen wird, dessen Ausgangssignal mit einem Tiefpassfilter (29a) gefiltert wird.

6. Verfahren nach Anspruch 5, **dadurch gekennzeichnet, dass** das Ausgangssignal des Drucksensors (28) zur Detektion eines Druckanstiegs oder Druckabfalls mit einem vorgegebenen oberen und/ oder unteren Grenzwert verglichen wird.

7. Vorrichtung zur extrakorporalen Blutbehandlung mit einem extrakorporalen Blutkreislauf (5A), der eine erste Kammer (3) eines durch eine Membran (2) in die erste Kammer und eine zweite Kammer (4) unterteilten Dialysators (1) oder Filters einschließt, und einem Flüssigkeitssystem (5B), das die zweite Kammer des Dialysators oder Filters einschließt, wobei eine Substitutionsflüssigkeitsleitung (17, 19, 20) zu dem Blutkreislauf stromauf oder stromab des Dialysators oder Filters führt, in der eine Substituatpumpe (22) angeordnet ist, und
einer Einrichtung zur Detektion der Zufuhr von Substitutionsflüssigkeit stromauf oder stromab des Dialysators oder Filters,
**dadurch gekennzeichnet, dass**
die Einrichtung zur Detektion der Zufuhr von Substitutionsflüssigkeit aufweist:

eine Steuereinheit (27) zum Abschalten und/ oder Einschalten der Substituatpumpe,
eine Messeinheit (28) zum Messen des Drucks im extrakorporalen Blutkreislauf (5) stromab des Dialysators (1) oder Filters, und eine
Auswerteinheit (29), die derart ausgebildet ist, dass eine Zufuhr von Substitutionsflüssigkeit stromauf oder stromab des Dialysators (1) oder Filters auf der Grundlage der Veränderung des Drucks nach dem Abschalten der eingeschalteten Substituatpumpe (22) und/oder dem Einschalten der abgeschalteten Substituatpumpe erkannt wird.

8. Vorrichtung nach Anspruch 7, **dadurch gekennzeichnet, dass**
die Steuereinheit (27) derart ausgebildet ist, dass für die Detektion der Zufuhr von Substitutionsflüssigkeit die eingeschaltete Substituatpumpe (22) abgeschaltet und nach Ablauf eines vorgegebenen Zeitintervalls die Substituatpumpe eingeschaltet wird, und die Auswerteinheit (29) derart ausgebildet ist, dass eine Zufuhr von Substitutionsflüssigkeit stromauf oder stromab des Dialysators (1) oder Filters auf der Grundlage des Wechsels zwischen einem vorausgehenden Druckanstieg und einem nachfolgenden Druckabfall oder zwischen einem vorausgehenden Druckabfall und einem nachfolgenden Druckanstieg erkannt wird.

9. Vorrichtung nach Anspruch 8, **dadurch gekennzeichnet, dass**
die Auswerteinheit (29) derart ausgebildet ist, dass auf die Zufuhr von Substitutionsflüssigkeit stromab des Dialysators (1) oder Filters geschlossen wird, wenn eine Abfolge von einem Druckanstieg und einem Druckabfall detektiert wird.

10. Vorrichtung nach Anspruch 8, **dadurch gekennzeichnet, dass** die Auswerteinheit (29) derart ausgebildet ist, dass auf die Zufuhr von Substitutionsflüssigkeit stromauf des Dialysators (1) oder Filters geschlossen wird, wenn eine Abfolge von einem Druckabfall und einem Druckanstieg detektiert wird.

11. Vorrichtung nach einem der Ansprüche 8 bis 10, **dadurch gekennzeichnet, dass** die Messeinheit zum Messen des Drucks im Blutkreislauf (5A) stromab des Dialysators (1) oder Filters einen Drucksensor (28) aufweist.

12. Vorrichtung nach einem der Ansprüche 8 bis 10, **dadurch gekennzeichnet, dass** die Auswerteinheit (29) einen Tiefpassfilter (29a) aufweist, der das Drucksignal des Drucksensors (28) empfängt, und einen Komparator (29b) aufweist, der das gefilterte Ausgangssignal des Drucksensors (28) zur Detektion eines Druckanstiegs oder Druckabfalls mit einem vorgegebenen oberen und/oder unteren Grenzwert vergleicht.

**Claims**

1. Method of monitoring the infeed of substitution fluid for an apparatus for extra-corporeal blood treatment having an extra-corporeal blood circuit (5A) which includes a first chamber (3) of a dialyser (1) or filter, which dialyser (1) or filter is divided by a membrane (2) into the first chamber and a second chamber (4), and having a fluid system (5B) which includes the second chamber of the dialyser or filter, substitution fluid being fed in upstream or downstream of the dialyser or filter by a substituate pump (22),
**characterised in that**,

for the detection of the infeed of substitution fluid, the substitute pump (22) is, when switched off, switched on and/or the substitute pump is, when switched on, switched off, and **in that** the pressure in the extra-corporeal blood circuit (5A) downstream of the dialyser (1) or filter is measured, an infeed of substitution fluid upstream or downstream of the dialyser or filter being detected on the basis of the change in pressure following the switching-off and/or switching-on of the substitute pump.

2. Method according to claim 1, **characterised in that**, for the detection of the infeed of substitution fluid, the substitute pump (22) is, when switched on, switched off and the substitute pump is switched on on expiry of a preset interval of time, and the pressure in the blood circuit (5A) downstream of the dialyser (1) or filter is measured, an infeed of substitution fluid upstream or downstream of the dialyser or filter being detected on the basis of the change between a preceding rise in pressure and a succeeding fall in pressure or between a preceding fall in pressure and a succeeding rise in pressure.

3. Method according to claim 2, **characterised in that** the infeed of substitution fluid downstream of the dialyser (1) or filter is deduced if a sequence of a rise in pressure and a fall in pressure is detected.

4. Method according to claim 2, **characterised in that** the infeed of substitution fluid upstream of the dialyser (1) or filter is deduced if a sequence of a fall in pressure and a rise in pressure is detected.

5. Method according to one of claims 1 to 4, **characterised in that** the pressure in the blood circuit (5A) downstream of the dialyser (1) or filter is measured by a pressure sensor (28) whose output signal is filtered by a low-pass filter (29a).

6. Method according to claim 5, **characterised in that**, for the detection of a rise in pressure or fall in pressure, the output signal from the pressure sensor (28) is compared with a preset upper and/or lower limiting value.

7. Apparatus for extra-corporeal blood treatment having an extra-corporeal blood circuit (5A) which includes a first chamber (3) of a dialyser (1) or filter, which dialyser (1) or filter is divided by a membrane (2) into the first chamber and a second chamber (4), and having a fluid system (5B) which includes the second chamber of the dialyser or filter, a line for substitution fluid (17, 19, 20), in which a substitute pump (22) is arranged, running to the blood circuit upstream or downstream of the dialyser or filter, and having an arrangement for detecting the infeed of substitution fluid upstream or downstream of the dialyser or filter,
**characterised in that**
the arrangement for detecting the infeed of substitution fluid has:

   a control unit (27) for switching off and/or switching on the substitute pump,
   a measuring unit (28) for measuring the pressure in the extra-corporeal blood circuit (5) downstream of the dialyser (1) or filter, and
   an analysing unit (29) which is so designed that an infeed of substitution fluid upstream or downstream of the dialyser (1) or filter is detected on the basis of the change in pressure following the switching-off of the substitute pump (22), when switched on, and/or the switching-on of the substituate pump, when switched off.

8. Apparatus according to claim 7, **characterised in that**
the control unit (27) is so designed that, for the detection of the infeed of substitution fluid, the substituate pump (22) is switched off, when switched on, and the substitute pump is switched on on expiry of a preset interval of time, and
the analysing unit (29) is so designed that an infeed of substitution fluid upstream or downstream of the dialyser (1) or filter is detected on the basis of the change between a preceding rise in pressure and a succeeding fall in pressure or between a preceding fall in pressure and a succeeding rise in pressure.

9. Apparatus according to claim 8, **characterised in that**
the analysing unit (29) is so designed that the infeed of substitution fluid downstream of the dailyser (1) or filter is deduced if a sequence of a rise and pressure and a fall in pressure is detected.

10. Apparatus according to claim 8, **characterised in that** the analysing unit (29) is so designed that the infeed of substitution fluid upstream of the dialyser (1) or filter is deduced if a sequence of a fall in pressure and a rise in pressure is detected.

11. Apparatus according to one of claims 8 to 10, **characterised in that** the measuring unit for measuring the pressure in the blood circuit (5A) downstream of the dialyser (1) or filter has a pressure sensor (28).

12. Apparatus according to one of claims 8 to 10, **characterised in that** the analysing unit (29) has a low-pass filter (29a) which receives the pressure signal from the pressure sensor (28), and a comparator (29b) which compares the filtered output signal from the pressure sensor (28) with a preset upper and/or lower limiting value to allow a rise in pressure or fall in pressure to be detected.

**Revendications**

1. Procédé de surveillance de l'apport de liquide de substitution pour un dispositif de traitement extracorporel du sang, comportant une circulation sanguine extracorporelle (5A) qui comprend une première chambre (3) d'un dialyseur (1) ou d'un filtre subdivisé par une membrane (2) en la première chambre et une deuxième chambre (4), et un système de liquide (5B) qui comprend la deuxième chambre du dialyseur ou du filtre, le liquide de substitution étant amené en amont ou en aval du dialyseur ou du filtre avec une pompe substitut,
   **caractérisé en ce que**,
   pour la détection de l'apport de liquide de substitution, la pompe substitut (22) arrêtée est mise en route et/ou la pompe substitut mise en route est arrêtée, et **en ce que** la pression est mesurée dans la circulation sanguine extracorporelle (5A) en aval du dialyseur (1) ou du filtre, un apport de liquide de substitution en amont ou en aval du dialyseur ou du filtre étant identifié sur la base de la modification de la pression après l'arrêt et/ou la mise en marche de la pompe substitut.

2. Procédé selon la revendication 1, **caractérisé en ce que**, pour la détection de l'apport du liquide de substitution, la pompe substitut mise en route (22) est arrêtée et après écoulement d'un laps de temps prédéterminé, la pompe substitut est mise en route et la pression dans la circulation sanguine (5A) est mesurée en aval du dialyseur (1) ou du filtre, un apport de liquide de substitution en amont ou en aval du dialyseur ou du filtre étant identifié sur la base de l'alternance entre une augmentation de pression antérieure et une chute de pression ultérieure ou entre une chute de pression antérieure et une augmentation de pression ultérieure.

3. Procédé selon la revendication 2, **caractérisé en ce que** l'apport du fluide de substitution en aval du dialyseur (1) ou du filtre permet de décider si une série d'une augmentation de pression et d'une chute de pression sera détectée.

4. Procédé selon la revendication 2, **caractérisé en ce que** l'apport du fluide de substitution en amont du dialyseur (1) ou du filtre permet de décider si une série d'une chute de pression et d'une augmentation de pression sera détectée.

5. Procédé selon l'une des revendications 1 à 4, **caractérisé en ce que** la pression dans la circulation sanguine (5A) est mesurée en aval du dialyseur (1) ou du filtre avec un capteur de pression (28) dont le signal de sortie est filtré avec un filtre passe-bas (29a).

6. Procédé selon la revendication 5, **caractérisé en ce que** le signal de sortie du capteur de pression (28) est comparé pour la détection d'une augmentation de pression ou d'une chute de pression, à une valeur seuil prédéterminée supérieure et/ou inférieure.

7. Dispositif de traitement sanguin extracorporel comprenant
   une circulation sanguine extracorporelle (5A) qui comprend une première chambre (3) d'un dialyseur (1) ou d'un filtre subdivisé par une membrane (2) en la première chambre et une deuxième chambre (4), et un système de liquide (5B) qui comprend la deuxième chambre du dialyseur ou du filtre, une conduite de liquide de substitution (17, 19, 20) conduisant à la circulation sanguine en amont ou en aval du dialyseur ou du filtre, dans laquelle une pompe substitut (22) est disposée,
   un dispositif de détection de l'apport du liquide de substitution en amont ou en aval du dialyseur ou du filtre, **caractérisé en ce que**
   le dispositif de détection de l'apport de fluide de substitution compren :

     une unité de commande (27) pour arrêter et/ou mettre en marche la pompe substitut,
     une unité de mesure (28) pour mesurer la pression dans la circulation sanguine extracorporelle (5) en aval du dialyseur (1) ou du filtre, et
     une unité d'analyse (29) qui est conçue de telle sorte qu'un apport du liquide de substitution en amont ou en aval du dialyseur (1) ou du filtre est identifié sur la base de la modification de la pression après l'arrêt de la pompe substitut (22) mise en marche et/ou la mise en marche de la pompe substitut arrêtée.

8. Dispositif selon la revendication 7, **caractérisé en ce que**
   l'unité de commande (27) est conçue de telle sorte que pour la détection de l'apport du liquide de substitution, la pompe substitut (22) mise en marche (22) est arrêtée et après écoulement d'un laps de temps prédéterminé, la pompe substitut est mise en route, et
   l'unité d'analyse (29) est conçue de telle sorte qu'un apport du liquide de substitution en amont ou en aval du dialyseur (1) ou du filtre soit identifié sur la base de l'alternance entre une augmentation de pression antérieure et une chute de pression ultérieure ou entre une chute de pression antérieure et une augmentation de pression ultérieure.

9. Dispositif selon la revendication 8, **caractérisé en**

**ce que**
l'unité d'analyse (29) est conçue de telle sorte qu'elle permette de décider en fonction de l'apport de liquide de substitution en aval du dialyseur (1) ou du filtre si une série d'une augmentation de pression et d'une chute de pression sera détectée.

10. Dispositif selon la revendication 8, **caractérisé en ce que** l'unité d'analyse (29) est conçue de telle sorte qu'elle permette de décider en fonction de l'apport de liquide de substitution en amont du dialyseur (1) ou du filtre si une série d'une chute de pression et d'une augmentation de pression sera détectée.

11. Dispositif selon l'une des revendications 8 à 10, **caractérisé en ce que** l'unité de mesure présente un capteur de pression (28) pour mesurer la pression dans la circulation sanguine (5A) en aval du dialyseur (1) ou du filtre.

12. Dispositif selon l'une des revendications 8 à 10, **caractérisé en ce que** l'unité d'analyse (29) présente un filtre passe-bas (29a) qui reçoit le signal de pression du capteur de pression (28) et présente un comparateur (29b) qui compare le signal de sortie filtré du capteur de pression (28) pour la détection d'une augmentation de pression ou d'une chute de pression, à une valeur seuil prédéterminée supérieure et/ou inférieure.

# Fig. 1

Fig. 2A

Fig. 2B

# Venenseitiger Druckverlauf während In Vitro HDF Dialysebehandlung

Dialysegerät: 4008   Uf-Rate: 0 ml/h   Blutfluss: 250 ml/min   Pre/Post Sub-Rate: 70 ml/min

**Fig. 3A** — HDF Post-Dilution

**Fig. 3B** — HDF Pre-Dilution

**EP 1 595 560 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0189561 A
- EP 1348458 A1
- DE 10115991 C1